(19) Europäisches Patentamt
European Patent Office
Office européen des brevets

(11) Numéro de publication: **0 124 502 B1**

(12) # FASCICULE DE BREVET EUROPEEN

(45) Date de publication de fascicule du brevet: **12.06.91**

(51) Int. Cl.[5]: **C07D 519/04**, A61K 31/475

(21) Numéro de dépôt: **84870057.1**

(22) Date de dépôt: **26.04.84**

Le dossier contient des informations techniques présentées postérieurement au dépôt de la demande et ne figurant pas dans le présent fascicule.

(54) **Nouveaux conjugués de la vinblastine et de ses dérivés, procédé pour leur préparation et compositions pharmaceutiques contenant ces conjugués.**

(30) Priorité: **29.04.83 LU 84784**
**29.12.83 LU 85161**

(43) Date de publication de la demande:
**07.11.84 Bulletin 84/45**

(45) Mention de la délivrance du brevet:
**12.06.91 Bulletin 91/24**

(84) Etats contractants désignés:
**AT BE CH DE FR GB IT LI LU NL SE**

(56) Documents cités:
**EP-A- 0 056 322**
**DE-A- 2 753 791**
**GB-A- 1 037 379**
**EP-A- 0 123 441**
**FR-A- 2 349 335**
**OA-A- 6 421**

**JOURNAL OF MEDICINAL CHEMISTRY, vol. 22, no. 4, 1979, pages 391-400, Easton, Pasadena, US; R.A. CONRAD et al.: "Structure-Activity Relationships of Dimeric Catharanthus Alkaloids. 2. Experimental Antitumor Activities of N-Substituted Deacetylvinblastine Amide (Vindesine) Sulfates"**

(73) Titulaire: **OMNICHEM Société anonyme**
**12 Avenue de Broqueville**
**B-1150 Bruxelles(BE)**

(72) Inventeur: **Trouet, André Benoît Léon**
**29, Predikherenberg**
**D-3009 Winksele(BE)**
Inventeur: **Rao, Kandukuri Sivaprasada Bushana**
**11, rue Kwakembienne**
**B-1331 Rosières(BE)**
Inventeur: **Hannart, Jean-Alfred Alphonse**
**25, avenue d'El Pirere**
**B-1302 Dion-Valmont(BE)**
Inventeur: **De Jonghe, Jean-Paul**
**94 rue Ch. Jaumotte**
**B-1350 Wavre(BE)**

(74) Mandataire: **Van Malderen, Michel et al**
**p.a. Freylinger & Associés 22 avenue J.S. Bach (bte 43)**
**B-1080 Bruxelles(BE)**

**EP 0 124 502 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention se rapporte à de nouveaux conjugués de la vinblastine et de certains de ses dérivés connus avec des protéines, servant d'agent anti-tumoral. L'invention s'étend également à un procédé pour les préparer et à des compositions pharmaceutiques contenant ces conjugués.

L'état de la technique est représenté par les documents suivants :

GB-A-1 037 379 (A)
EP-A-0 123 441 (B)
J. Med. Chem. 22, 391 (1979) (C)
EP-A-0 056 322 (D)
OA-A-6 421 (E)
FR-A-2 349 335 (F)

Ces documents décrivent divers dérivés de la vinblastine (VLB) et rapportent leurs propriétés anti-cancéreuses.

Le problème qui ressort de cet état de la technique est de mettre au point des dérivés conjugués de la VLB ayant des propriétés antitumorales de même niveau ou de niveau supérieur par rapport à l'activité des molécules connues.

Les composés de l'invention sont ceux indiqués à la revendication 1.

Dans l'art antérieur, la liaison était effectuée entre-autres par l'intermédiaire d'un lien amide au niveau de la fonction vinblastinoyl-23 du dérivé bis-indolique. De manière surprenante il a été constaté que l'activité anti-tumorale peut être mieux conservée lorsque la liaison est effectuée en C-4 plutôt qu'en C-23.

Les dérivés de la présente invention sont obtenus, dans une première étape, par condensation de l'anhydride chloroacétique, sur l'hydroxyle en C-4 de la vindésine, de la O-4 désacétylvinblastine ou de la O-4 désacétylvincristine.

Le dérivé,hémisuccinate ainsi obtenu est alors condensé avec de l'albumine humaine galactosylée dans un solvant dans lequel ces composés sont solubles. On peut à cette fin utiliser un mélange eau-dioxanne à un pH adéquat maintenu par un tampon , par exemple un tampon borate. La condensation est confirmée par chromatographie ou électrophorèse. Dans ce dernier cas on peut utiliser de la vinblastine radio-active (par exemple tritiée) afin de faciliter la caractérisation.

Du point de vue chimique la condensation avec les protéines peut s'expliquer par l'obtention de liens covalents résultant de la réaction des groupes amines de la lysine protéique avec le groupe ester activé dérivé de l'hémisuccinate.

L'albumine de sérum bovin par exemple comprend un nombre élevé résidus aminés de lysine. Le nombre de molécule de vinblastine par protéine variera en fonction des conditions opératoires mais sera généralement compris entre 1 et 34.

L'activation peut être effectuée de manière classique par traitement avec un chloroformiate d'alkyle, de préférence le chloroformiate d'éthyle ou d'isobutyle, en présence d'une base aminée telle la N-méthyl pipéridine ou la N-méthyl morpholine.

La condensation peut être effectuée in situ sur le mélange réactionnel contenant l'anhydride activé. Dans la plupart des cas l'anhydride activé peut cependant aussi être isolé.

Le conjugué obtenu est isolé au moyen de méthodes classiques utilisées en chimie ou, dans le cas de conjugués protéiques, en biochimie. Le conjugué protéique est ainsi précipité du milieu réactionnel par addition d'acétone, puis centrifugé, rincé, lyophilisé et purifié par filtration sur gel. Eventuellement on peut soumettre le dérivé ainsi obtenu à une réaction de succinylation classique qui permet d'éviter des problèmes d'agrégation que caractérisent certaines protéines, conjuguées ou non.

La protéine utilisée est l'albumine humaine traitée afin d'être sélectivement modifiée. Cette modification permet d'obtenir des conjugués protéiques qui seront, lors de leur utilisation thérapeutique préférentiellement concentrés au niveau de certains tissus, par exemple au niveau du foie. Cette modification est une galactosylation effectuée en appliquant par exemple le mode opératoire décrit par G.Wilson dans The Journal of Biochemistry , 253 (7) 2070-2072, 1978.

Les essais in-vitro effectués avec les composés de l'invention pour démontrer leur activité anti-tumorale indiquent que la formation du conjugué par un lien en C-4 est plus avantageuse que lorsque ce lien est effectué en C-3.

Exemple 1 (REFERENCE)
Vindésine-0-4-hémisuccinate et couplage avec l'albumine humaine (AH) - (AH-Succ-VDS)

Dans un ballon, on introduit 0.28 mmol (210 mg) de vindésine, 42 mg (0.4 mmol) d'anhydride succinique, et 6 mL de $CH_2Cl_2$ anhydre. La solution est agitée à l'abri de la lumière à température ambiante pendant 14h. Le solvant est évaporé. Le résidu est repris dans 4,7 mL de dioxanne. La solution est ensuite plongée dans un bain de glace. On y ajoute 0,56 mL de triéthylamine dans 3,5 mL de dioxanne et 0,56 mL de chloroformiate d'isobutyle dans 3,5 mL de dioxanne. On agite pendant 1h. D'autre part on prépare une solution de 208 mg de AH dans 38 mL d'$H_2O$ et 26 mL de dioxanne y sont ajoutés goutte à goutte. Le pH est ajusté à 8,5 avec de la soude 1N. La solution est refroidie à 5° C. Après 1 h 00, l'ester activé est ajouté à la solution d'albumine humaine. La solution est agitée pendant 14h. à 5° C tandis que le pH est maintenu à 8.5 par addition de NaOH 1N. La précipitation du conjugué et sa purification sont effectués comme précédemment décrits. Le rapport molaire est de 13,8.

Exemple 2. (SELON L'INVENTION)
Vindésine-0-4-hémisuccinate et couplage avec l'albumine humaine galactosylée (AHgal) (AHgal-Succ-VDS).

Dans un ballon, on introduit 0.16 mmol (119 mg) de vindésine, 0,24 mmol (24 mg) d'anhydride succinique et 3,4 mL de $CH_2Cl_2$ anhydre. La solution est agitée à l'abri de la lumière à température ambiante pendant 14 h. Le solvant est évaporé. Le résidu est repris dans 2,7 mL de dioxanne. La solution est ensuite plongée dans un bain de glace. On y ajoute 0,32 mmol de triéthylamine dans 2 mL de dioxanne et 0,32 mmol de chloroformiate d'isobutyle dans 2 ml de dioxanne. On agite pendant 1h. D'autre part, on prépare une solution de 164 mg d'AHgal dans 17.5 mL d'$H_2O$ et 25 mL de dioxanne y sont ajoutés goutte à goutte. Le pH est ajusté à 8,5 avec de la soude 1N. La solution est refroidie à 5° C. Après 1h., l'ester activé est ajouté à la solution d'AHgal. La solution est agitée pendant 14 h à 5° C. tandis que le pH est maintenu à 8,5 avec de la soude 1N. La précipitation du conjugué et sa purification sont effectués comme précédemment décrits. Le rapport molaire est de 21,6.

Les composés de l'invention ont été testés sur des souris $BDF_1$ ou $DBA_2$ auxquelles une leucémie P388 a été implantée par voie intra - péritonéale ou intra-veineuse, l'activité anti-tumorale étant mesurée par le pourcentage d'ILS (Increased Life Span, augmentation de la durée de survie ).

Ainsi des expériences effectuées avec la vindésine, le N-(04-déacétyl-3-de (méthoxycarbonyl)-vinblastinoyl-23) tryptophanate d'éthyle (voir demande de brevet européen Publ. n° 41.935, Omnichem), ainsi que d'autres dérivés en C-4 d'acides aminés, couplé via le carbone C-3 avec la fétuine ou l'albumine de sérum bovin dans un rapport variant de 2 à 6, démontrent que ces conjugués sont peu actifs sur les tumeurs P388.

L'administration du oonjugué a été effectuée par voie intra-péritonéale ou intra-veineuse, identique au mode d'injection de la tumeur.

Par contre ces mêmes substances pour lesquels le couplage est effectué en C-4 montrent, de manière inattendue, une activité significative à l'occasion de tests similaires. Des augmentations de durée de survie de plus de 70% ont été observées.

L'activité supérieure des conjugués protéiques de la présente invention a également été mise en évidence sur des cellules L1210 in vitro en milieu RPMI contenant 10% de sérum de veau foetal. Après incubation, la technique adoptée consiste à mesurer le contenu en protéines cellulaires, estimation effectuée en adoptant la technique de Lowry. Ainsi après 3 jours à une concentration de 10-50 microgrammes/mL de BSA(S)-C4-VDS on obtient une inhibition de la croissance cellulaire, alors que la culture cellulaire non traitée s'est multipliée par un facteur 2,5.

Des cellules de l'hépatome Hep. G2 (300.000 céllules) ont été incubées pendant 8 jours en présence d'AHgal - VDS-C-4 et d'AHgal-VDS-C-3 à des concentrations variant de 1,3 à 21,700 ng de VDS/ml. Après 8 jours , le taux de protéines cellulaires est estimé par la technique de Lowry.
Les résultats montrent que la concentration en drogue tuant 50% des cellules est >1.000ng/ml dans le cas de l'AHgal-VDS-$C_3$ et est de 215ng/ml dans le cas de l'AHgal-VDS-$C_4$.

TABLEAU

ACTIVITE DU AHgal-Succ-VDS POUR DES TUMEURS INDUITES CHEZ LA SOURIS.

| PRODUIT | (a) | (b) | (c) | (d) | (e) | (f) | (g) | (h) |
|---|---|---|---|---|---|---|---|---|
| A Hgal-Succ-VDS | 21,6 | 75 | 356 | $BDF_1$ | $10^6$ | P388 | ip | 57 |

(a) rapport molaire vinca:protéine
(b) dose mg vinca/kg
(c) dose mg protéine/kg
(d) souche de souris
(e) nombre de cellules injectées
(f) type de la tumeur injectée
(g) voie d'injection
(h) pourcentage d'augmentation du temps de survie des survivants comparativement à des souris non-traitées (ILS).

La présente invention s'étend donc également aux applications industrielles et notamment pharmaceutiques de composés bisindoliques tels que revendiqués dans la présente demande.

Les composés de l'invention présentent en effet des propriétés antitumorales particulièrement intéressantes et susceptibles d'être utilisées en thérapeutique humaine.

Ces dérivés de la vinblastine peuvent être , en particulier, utilisés pour le traitement des leucémies, de gliomes, de lymphosarcomes ou d'autres tumeurs malignes, y compris les tumeurs dites "solides".

En thérapeutique humaine , ils sont ainsi utilisés pour le traitement de la maladie de Hodgkin et pour d'autres tumeurs pouvant bénéficier d'un traitement avec la vinblastine, la vincristine ou la vindésine.

Pour leur application thérapeutique, les composés de l'invention, éventuellement sous forme lyophilisée, sont de préférence administrés par voie parentérale, dissous dans un solvant pharmaceutiquement acceptable. Parmi les sels d'addition, on préférera, les sels non toxiques pharmaceutiquement acceptables, tels que les sels d'acides minéraux comme l'acide chlorhydrique, l'acide phosphorique et l'acide sulfurique ou d'acides organiques comme l'acide acétique, propionique, succinique, tartrique, oxalique, méthanesulfonique ou benzènesulfonique.

L'eau physiologique ou d'autres solutions salines tamponnées, par exemple avec un phosphate, sont des solvants appropriés.

La substance active est généralement administrée à une posologie pouvant varier de 50 mg à plusieurs grammes . Les composés de l'invention peuvent en outre être utilisés en combinaison avec d'autres agents anti-tumoraux.

## Revendications

1. Conjugué de vindésine, désacétylvinblastine ou de désacétylvincristine caractérisé en ce qu'une albumine humaine galactosylée est liée à la vinblastine ou à un dérivé de celle-ci en $C_4$ via un lien

$-CO-(CH_2)_2-CO-$de manière à former un composé de formule

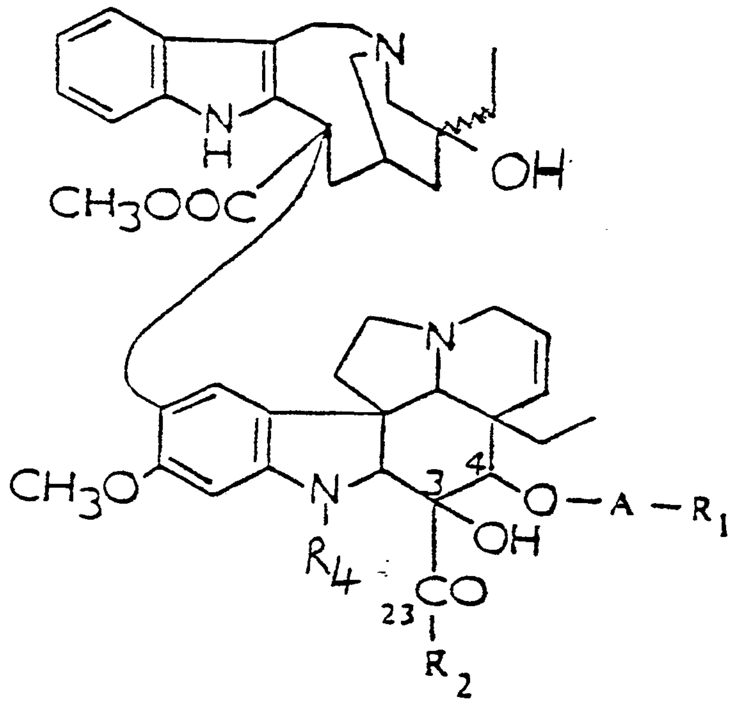

dans laquelle A représente le lien $-CO(CH_2)_2-CO-$,
$R_1$ représente un reste d'albumine humaine galactosylée,
$R_2$ représente un groupe méthoxy ou un groupe amino,
$R_4$ représente un reste formyle pour les conjugués désacétyl-vincristine ou un groupe méthyle pour les conjugués de vindésine et désacétyl-vinblastine, ainsi que leurs sels d'addition avec un acide minéral ou organique.

2. Procédé de préparation de conjugués suivant la revendication 1, caractérisé en ce qu'on condense, dans une première étape, l'anhydride succinique sur l'hydroxyle en position C-4 de la vindésine, de la O-4-désacétylvinblastine ou de la O-4-désacétylvincristine et en ce qu'on condense ensuite les dérivés ainsi obtenus avec de l'albumine humaine galactosylée dans un solvant dans lequel ces composés sont solubles.

3. Procédé suivant la revendication 2 caractérisé en ce que le solvant utilisé dans la deuxième étape de condensation consiste en un mélange eau-dioxanne à un pH adéquat maintenu par un tampon.

4. Procédé suivant l'une quelconque des revendications 2 ou 3 caractérisé en ce qu'on isole le conjugué obtenu par précipitation du milieu réactionnel et en ce qu'on effectue encore une centrifugation, un rinçage, une lyophilisation et une purification par filtration sur gel, ainsi qu'une succinylation classique.

5. Composition pharmaceutique caractérisée en ce qu'elle comporte des conjugués selon la revendication 1 en combinaison avec un diluant, solvant, excipient ou support pharmaceutiquement acceptable.

6. Composition pharmaceutique selon la revendication 5 caractérisée en ce que le composé actif se trouve sous forme lyophilisée.

7. Composition pharmaceutique selon la revendication 5 caractérisée en ce que le composé actif est en solution dans un solvant tamponné.

**Claims**

1. Conjugate of vindesine, deacetylvinblastine or deacetylvincristine characterized in that a human galactosylated albumin is bonded to the vinblastin or to a derivative thereof in $C_4$ via a bond $-CO-(CH_2)_2-CO$ in such a way to form a compound of the formula

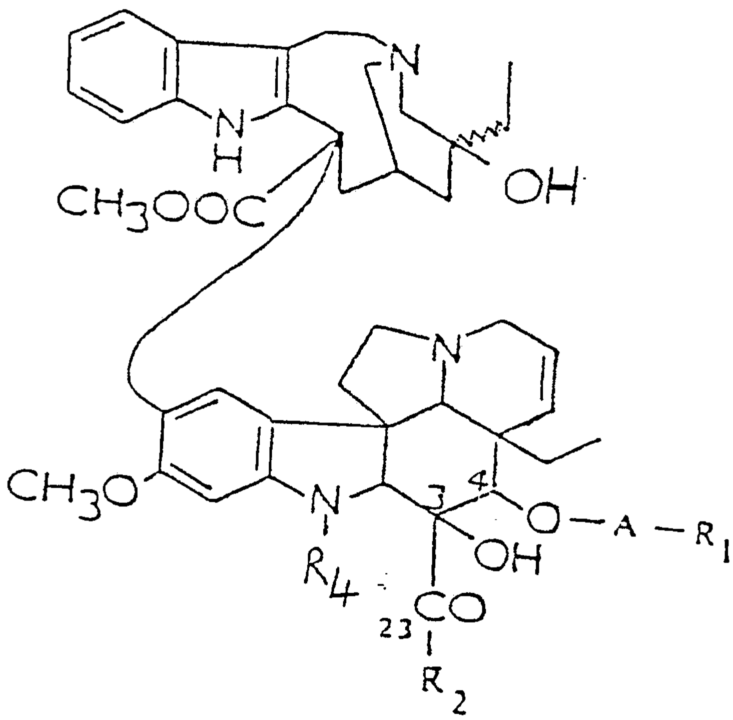

wherein A represents the bond $-CO(CH_2)_2-CO-$
$R_1$ represents a moiety of human galactosylated albumin,
$R_2$ represents a methoxy or amino group,
$R_4$ represents a formyl moiety for the deacetylvincristine conjugates or a methyl group for the vindesine and deacetylvinblastine conjugates,
and their addition salts with a mineral or organic acid.

2. Process for preparing conjugates according to claim 1, characterized in that one condenses in a first stage, the succinic anhydride onto the hydroxyl at C-4 position of the vindesine, the 0-4-deacetylvinblastine or the O-4-deacetylvincristine and in that one condenses further the derivatives so obtained with human galactosylated albumin in a solvent in which these compounds are soluble.

3. Process according to claim 2, characterized in that the solvent used in the second condensation step consists in a mixture water-dioxane at a suitable pH maintained by a buffer.

4. Process according to any one of claims 2 or 3, characterized in that one isolates the obtained conjugate by precipitation of the reaction medium and in that one carries out again a centrifugation, a rinsing, a lyophilization and a purification by gel filtration, as well as a conventional succinylation.

5. Pharmaceutical composition characterized in that it comprises conjugates according to claim 1 in combination with a diluent, solvent, carrier or support pharmaceutically acceptable.

6. Pharmaceutical composition according to claim 5, characterized in that the active compound is under lyophilized form.

7. Pharmaceutical composition according to claim 5, characterized in that the active compound is in solution in a buffered solvent.

**Ansprüche**

1. Vindesin-, Deacetylvinblastin- oder Deacetylvinkristin-Konjugat, dadurch gekennzeichnet, daß ein galaktosiliertes menschliches Albumin mit dem Vinblastin oder einem seiner Derivate von $C_4$ über ein Bindeglied $-CO(CH_2)_2-CO-$ so verbunden wird, daß eine Verbindung gemäß Formel

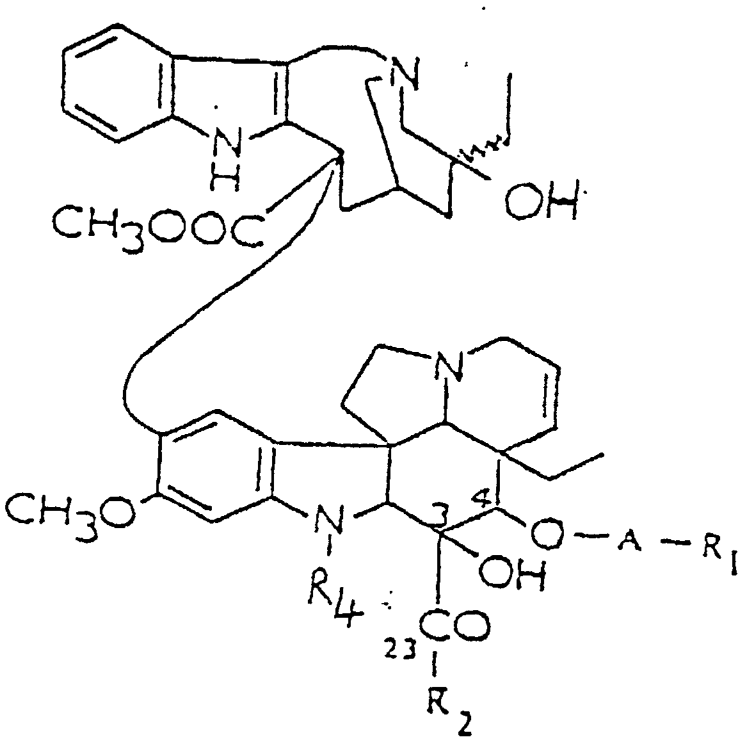

entsteht, in der A das Bindeglied $-CO(CH_2)_2$ -CO- ist, $R_1$ ein Rest des galaktosilierten menschlichen Albumins, $R_2$ eine Methoxygruppe oder eine Aminogruppe, $R_4$ ein Rest an Formyl für die Deacetylvinkristin-Konjugate oder eine Methylgruppe für die Vindesin- und Deacetylvinblastin-Konjugate sowie ihre Zusatzsalze mit einer Mineralsäure oder organischen Säure istt.

2. Verfahren für die Gewinnung der Konjugate nach Anspruch 1, dadurch gekennzeichnet, daß in einer ersten Stufe das Sukzinsäureanhydrid auf dem Hydroxyl in Position C-4 des Vindesin, des 0-4-Deacetylvinblastin oder des 0-4-Deacetylvinkristin kondensiert wird und daß anschließend die somit gewonnenen Derivate mit den galaktosilierten menschlichen Albumin in einer Lösung kondensiert werden, in der diese chemischen Verbindungen löslich sind.

3. Verfahren nach Anspruch 2, dadurch gekennzeichnet, daß die für die zweite Kondensationsstufe verwendete Lösung aus einer Mischung aus Wasser und Dioxan mit geeignetem pH-Wert besteht, der mittels Puffer aufrechterhalten wird.

4. Verfahren nach Anspruch 2 oder 3, dadurch gekennzeichnet, daß das durch Ausfällen des Reaktionsmediums gewonnene Konjugat isoliert wird und daß noch eine Zentrifugierung, eine Spülung, eine Lyophilisation und eine Reinigung mittels Gelfiltration sowie eine herkömmliche Sukzinylation erfolgen.

5. Pharmazeutisches Präparat, dadurch gekennzeichnet, daß es die Konjugate nach Anspruch 1 in Verbindung mit einem Verdünnungsmittel, einem Lösungsmittel und einem pharmazeutisch verträglichen Trägerstoff oder Stützsubstanz enthält.

6. Pharmazeutisches Präparat nach Anspruch 5, dadurch gekennzeichnet, daß die aktive Substanz sich in gefriergetrocknetem Zustand befindet.

7. Pharmazeutisches Präparat nach Anspruch 5, dadurch gekennzeichnet, daß die aktive Substanz in einem gepufferten Lösungsmittel gelöst ist.